# FASCICULE DE BREVET EUROPEEN

(11) **EP 0 884 521 B1**
(45) Date de publication et mention de la délivrance du brevet: **16.04.2008**
(21) Numéro de dépôt: 98401156.9
(22) Date de dépôt: 14.05.1998
(51) Int. Cl.: F17C 13/04, F16K 17/04, F16K 17/16

(54) **Ensemble d'alimentation de gaz destiné à être raccordé à un réservoir contenant un gaz sous haute pression**
Gasversorgungseinheit zum Anschluss an einen Hochdruckgasbehälter
Gas supplying assembly for connection to a high pressure gas container

(30) Priorité: 15.05.1997 FR 9705961
(43) Date de publication de la demande: 16.12.1998
(73) Titulaire: DELEGATION GENERALE POUR L'ARMEMENT, BUREAU DE LA PROPRIETE INDUSTRIELLE D.R.E.T., 00460 Armées (FR); Sperian Respiratory Protection France, 93420 Villepinte (FR)
(72) Inventeur: Beauvais, Vincent, 91690 Fontaine la Rivière (FR); Evrard, Patrick, 45000 Orléans (FR)

(56) Documents cités:
- EP-A- 0 588 531
- FR-A- 2 706 052
- GB-A- 2 111 154
- US-A- 4 256 138

## Description

La présente invention concerne un ensemble d'alimentation de gaz, prévu pour équiper un réservoir rempli de gaz sous haute pression, et comportant un robinet d'isolement, un détendeur basse pression et des sorties basse pression. Elle s'étend aussi aux bouteilles de gaz équipées de ce type d'ensemble.

Le secteur technique de l'invention est celui de la fourniture des gaz médicaux, ou industriels sous basse pression destinés au fonctionnement de matériels divers ou à l'oxygénothérapie, plus particulièrement appliqué au milieu aéronautique.

On connaît des dispositifs raccordés sur des réservoirs de gaz sous pression permettant le passage de la haute pression à la basse pression.

Ces dispositifs peuvent se présenter sous différentes configurations :
- intégrés directement ou non au réservoir ou à la bouteille haute pression,
- avec ou sans robinet permettant l'interruption du débit de gaz,
- avec ou sans débitmètre ou manomètre.

Dans le cas des évacuations sanitaires aériennes de blessés, le transport et l'utilisation d'oxygène médical, tant sur aéronefs civils que militaires, sont soumis à des conditions particulières de manière à assurer la sécurité en vol.

En outre des conditions mécaniques et climatiques sévères sont à prendre en considération pour un bon fonctionnement des dispositifs médicaux.

Des appareils connus du type détendeur à piston permettent d'abaisser la haute pression jusqu'à un niveau compatible à celui nécessaire aux équipements alimentés.

Le brevet FR 2 706 052, considéré comme représentant l'art antérieur le plus proche, décrit un ensemble de commande et de distribution de gaz destiné à être intégré sur une bouteille de gaz médicaux ou non.

Néanmoins ce matériel présente les inconvénients propres aux détendeurs classiques à piston :
- fonctionnement non étudié dans le cas de très basses températures,
- problèmes liés à un environnement vibratoire sévère (cas des aéronefs à hélice notamment), pouvant nuire au bon fonctionnement de la régulation du détendeur,
- non respect des spécifications aéronautiques, si la bouteille équipée fait partie intégrante de l'équipement de l'aéronef,
- non étanchéité de l'ensemble à une pollution de type nucléaire, bactériologique ou chimique (NBC)

On connaît également le brevet FR-A-2 706 052, document reflétant l'état de la technique le plus proche, qui décrit un régulateur pour gaz comprimé hautes pressions pour bouteille de gaz comportant deux dispositifs de sécurité. L'un de ces dispositifs comprend un clapet muni de deux joints circulaires faisant étanchéité dans une chambre, l'autre dispositif comportant un piston également muni d'un joint d'étanchéité placé dans une gorge coulissant dans un couvercle.

Ce régulateur présente certains des inconvénients énumérés ci-dessus, notamment il met en, oeuvre des éléments mobiles (clapet, piston) inadaptés aux basses températures et présentant des forces de frottement importantes, ce qui le rend inutilisable en milieu aéronautique.

La présente invention a pour but de disposer de ce type d'ensemble compact et indéréglable installé en permanence sur un réservoir de gaz, dans le cas notamment d'utilisation dans des conditions d'environnement climatiques sévères, et pendant des phases de transports aériens, maritimes, ferroviaires ou routiers, tout en garantissant les performances demandées par l'utilisateur et la sécurité de son environnement.

Pour ce faire, l'invention a pour objet un ensemble d'alimentation de gaz destiné à être raccordé à un réservoir contenant un gaz sous haute pression, comprenant une partie basse pression fixée sur une partie haute pression, chaque partie comportant un dispositif de sécurité.

Cet ensemble d'alimentation de gaz se caractérise en ce que ces deux dispositifs de sécurité sont reliés entre eux par un collecteur d'échappement à sortie unique, canalisant le gaz vers l'extérieur de l'ensemble d'alimentation.

De préférence, la partie basse pression comporte un piston creux à soufflet métallique déformable, mobile à faible frottement sur une buse de la partie haute pression.

L'ensemble d'alimentation selon la présente invention peut comporter un conduit, pratiqué dans le corps de la partie haute pression, mettant l'intérieur du piston à la pression atmosphérique.

Selon un mode de réalisation préférentiel, un ressort de compression, placé à l'intérieur du piston, équilibre l'effet de la pression du gaz pour la fermeture de la buse par un clapet disposé dans la tête du piston.

De préférence, le dispositif de sécurité haute pression comprend un disque de rupture dont l'éclatement calibré libère le gaz provenant de la bouteille par le collecteur d'échappement à sortie unique.

De préférence, le dispositif de sécurité basse pression comprend un clapet taré se déplaçant sous l'effet d'une surpression pour permettre l'évacuation du gaz à travers le collecteur à sortie unique.

L'ensemble d'alimentation peut comporter un clapet, placé en amont du dispositif de sécurité haute pression, limitant la pression résiduelle de la bouteille à un niveau supérieur à celui de la pression de l'air ambiant.

La partie haute pression peut comporter un robinet commandant le passage, dans la buse, du gaz provenant de la bouteille.

Le robinet, muni d'un volant, comporte un doigt coulissant dans un logement du volant pour indiquer l'ouverture ou la fermeture du robinet, selon la position rentrée ou sortie du doigt dans le logement.

De préférence, les matériaux constitutifs de cet ensemble sont compatibles aux contraintes liées à l'oxygène sous haute pression, à la non génération de gaz toxiques en cas d'inflammation, ainsi qu'aux contraintes liées à l'utilisation en milieu aéronautique.

Grâce à son collecteur d'échappement à sortie unique, cet ensemble d'alimentation présente l'avantage de faciliter l'évacuation à l'extérieur de l'aéronef, du gaz provenant du fonctionnement de l'un des dispositifs de sécurité basse ou haute pression.

Ceci est particulièrement important pour éviter toute diffusion accidentelle du gaz et participer notablement à limiter l'encombrement à bord d'un aéronef.

Un autre avantage réside dans l'utilisation d'un piston à soufflet métallique déformable mobile sans frottement, assurant une très bonne fiabilité de fonctionnement indépendamment de la température extérieure.

Cet ensemble d'alimentation présente également l'avantage d'être très étanche et totalement adapté aux contraintes sévères et aux exigences spécifiques aux conditions d'utilisation et de sécurité, rencontrées notamment en milieu aéronautique.

Un exemple de réalisation, illustré en trois vues, est donné ci-après et permet de montrer les détails et d'autres avantages de l'invention. Il est simplement fourni à titre indicatif mais non limitatif, l'invention pouvant prendre d'autres configurations de conception.

La figure 1 montre une vue de dessus d'un ensemble d'alimentation de gaz selon la présente invention.

La figure 2 représente une vue en coupe longitudinale de l'ensemble d'alimentation, selon II - II de la figure 1.

La figure 3 représente également une vue en coupe longitudinale de l'ensemble d'alimentation, selon III - III de la figure 1.

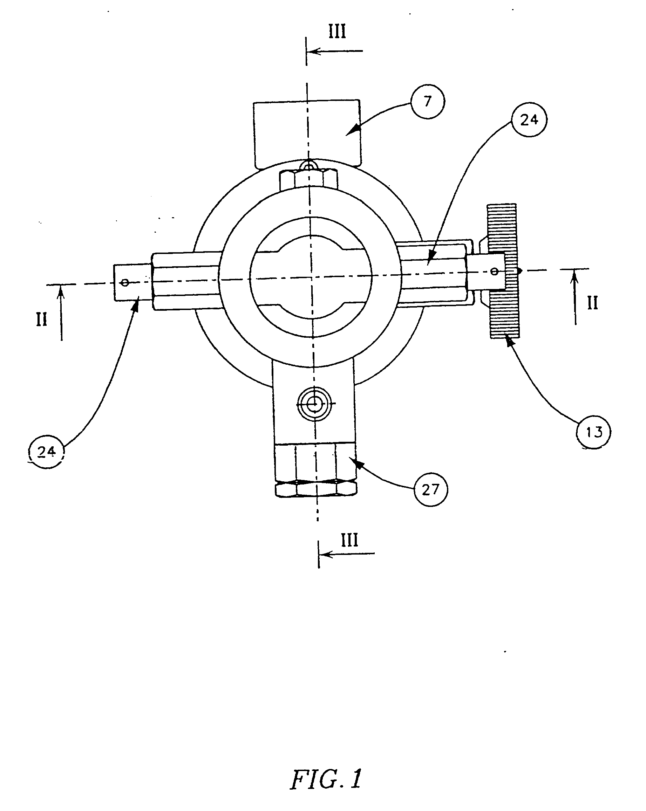

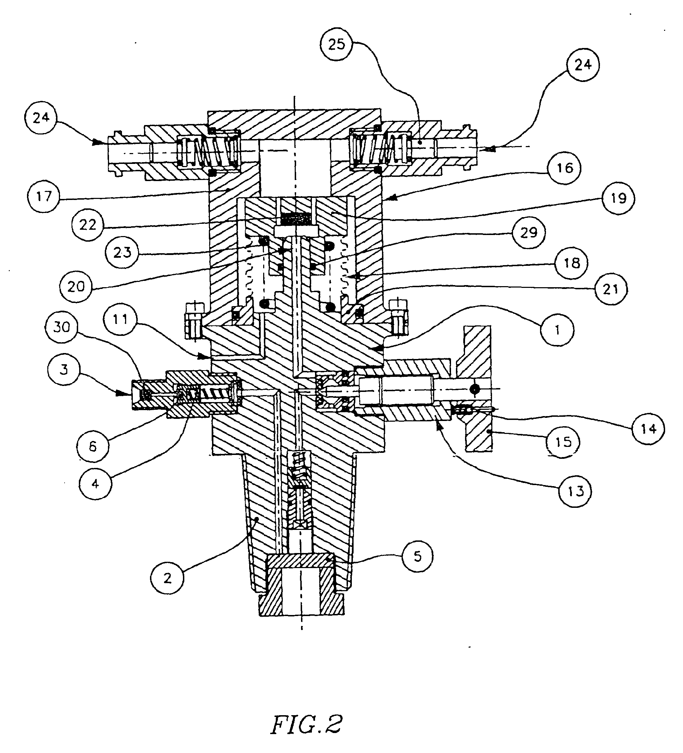

En référence aux figures 1 à 3, on voit un ensemble d'alimentation selon la présente invention, comprenant une partie haute pression 1 comportant un corps 2 dont l'extrémité inférieure est filetée pour se visser sur un goulot d'une bouteille de gaz (non représentée), ici considérée comme remplie d'oxygène médical.

Le fonctionnement des divers éléments constitutifs de la partie haute pression 1, est défini ci-après.

Le chargement de la bouteille se fait par l'intermédiaire du raccord de remplissage 3, comportant un clapet anti-retour 4 monté en aval d'un filtre 30 en bronze fritté permettant, lors des remplissages, d'absorber au passage une partie des calories du gaz pour préserver un joint 6 du clapet anti-retour 4.

Si un phénomène d'ignition, lié aux risques de compression adiabatique encourus lors des remplissages en oxygène, intervient, la propagation de la chaleur liée à une éventuelle combustion du joint 6 du clapet anti-retour 4 se limitera au filtre 30, dont la masse permet d'absorber la chaleur et de préserver l'intérieur de la bouteille et le reste de l'ensemble d'alimentation.

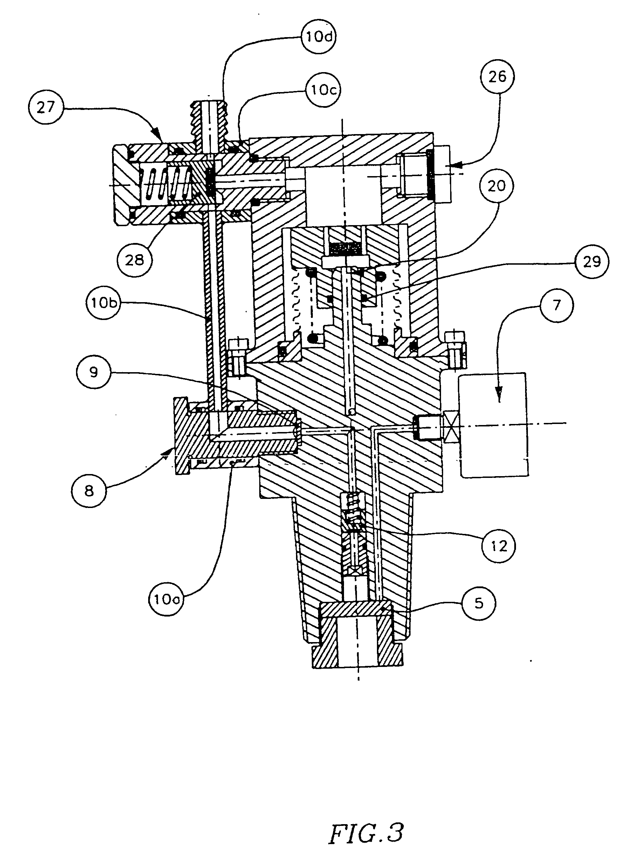

La pression de l'oxygène contenu dans la bouteille est lue en permanence sur un manomètre 7 (figure 3).

Dans le cas d'une montée accidentelle de la pression interne de la bouteille, un dispositif de sécurité haute pression 8 comportant un disque de rupture 9, dont l'éclatement calibré n'excède pas la pression d'épreuve de la bouteille, libère le gaz vers l'extérieur au travers d'un collecteur d'échappement 10. Celui-ci est constitué des pièces 10a, 10b, 10c et 10d.

L'extrémité haute pression 10a est reliée par un tube 10b à l'extrémité basse pression 10c, laquelle comporte une sortie unique 10d. Ceci permet de canaliser vers l'extérieur la pression excédentaire, pour ne pas augmenter la concentration d'oxygène de l'air environnant et risquer une explosion, notamment dans le cas d'utilisation à bord d'aéronefs, ou en milieu confiné sensible.

Dans tous les cas de vidange de la bouteille, que ce soit accidentel comme vu précédemment ou normal en consommant l'oxygène administré au travers d'un filtre 5 en bronze fritté, un clapet taré 12 monté en amont du dispositif de sécurité haute pression 8 et d'un robinet 13, limite la pression résiduelle de la bouteille à un niveau toujours supérieur à celui de la pression extérieure, et ceci quelle que soit l'altitude d'utilisation de l'ensemble d'alimentation.

L'ouverture du robinet 13 conditionne l'utilisation du gaz contenu dans la bouteille.

Un doigt 14, coulissant dans un logement d'un volant 15 du robinet 13, permet de vérifier rapidement au toucher l'état du robinet : doigt complètement ressorti signifie fermeture, et rentré ouverture.

Sur la partie haute pression 1, se trouve fixée, par vis ou tout autre moyen équivalent, une partie basse pression 16 dont l'enveloppe externe est matérialisée par un corps creux 17.

La cavité interne de ce corps creux 17 constitue la chambre basse pression.

A l'intérieur de celle-ci, se trouve un détendeur constitué d'un piston 18 creux à soufflet métallique déformable dont la tête 19 coulisse de façon étanche sur une buse 20, appartenant à la partie haute pression 1, et dont une partie inférieure 21 du piston est bridée de façon étanche entre le corps 2 de la partie haute pression 1 et celui 17 de la partie basse pression 16.

Ce piston déformable 18 constitue l'élément comparateur du détendeur.

L'intérieur du piston 18 étant à la pression atmosphérique par l'intermédiaire d'un conduit 11 pratiqué dans le corps 2, le soufflet se comprime et occasionne l'obturation de la buse 20 par un clapet 22 disposé dans la tête 19 du piston, sous l'effet de la pression du gaz contenu dans la chambre basse pression.

La chambre basse pression est alimentée par la buse 20, elle-même alimentée en gaz par le robinet 13.

Le niveau de pression de cette chambre, occasionnant la fermeture de la buse, est principalement conditionné par la réaction d'un ressort de compression 23 placé à l'intérieur du piston 18, ce ressort de compression 23 étant choisi judicieusement pour obtenir la basse pression régulée souhaitée.

Ce type de détendeur à soufflet métallique permet de garantir une bonne stabilité de la pression régulée en fonction de la température extérieure, car la souplesse du soufflet n'en est pratiquement pas affectée et le frottement de glissement d'un joint torique 29, assurant l'étanchéité dynamique entre la tête 19 du piston 18 et la buse 20 du corps 2 est négligeable vu sa petite taille.

De plus, le clapet 22 étant solidaire de la tête 19 du piston 18, le détendeur tente de rechercher l'étanchéité de sa fermeture, ce qui est un point positif au niveau sécurité.

En aval du détendeur et à la partie supérieure du corps 17, on dispose de deux sorties basse pression 24 avec clapet anti-retour 25.

On dispose également d'une troisième sortie, qui est obturée par un bouchon 26 lorsque celle-ci n'est pas utilisée.

Un dispositif de sécurité basse pression 27 protège la partie basse pression 16 d'une surpression accidentelle.

Le gaz en excès repousse un clapet taré 28 pour s'échapper par l'extrémité basse pression 10c et la sortie unique 10d, afin de préserver l'environnement immédiat des risques d'air suroxygéné.

Lors d'une application "oxygène médical", les pièces mécaniques de l'ensemble d'alimentation sont réalisées dans des alliages de cuivre ou de matériaux inoxydables, de façon à assurer une bonne tenue de l'ensemble lors des chargements en gaz. Les matières plastiques et élastomères sont choisies de façon à ne pas dégager de gaz toxiques en cas d'inflammation.

Une version de l'invention peut facilement être faite pour garantir une absence de pollution du gaz distribué dans le cas d'un environnement contaminé nucléaire, bactériologique ou chimique (NBC).

## Revendications

1. - Ensemble d'alimentation de gaz destiné à être raccordé à un réservoir contenant un gaz sous haute pression, comprenant une partie basse pression (16) contenant un piston (18) et étant fixée sur une partie haute pression (1), chaque partie comportant un dispositif de sécurité (27, 8) et ces deux dispositifs de sécurité étant reliés entre eux par un collecteur d'échappement (10) à sortie unique canalisant le gaz vers l'extérieur de l'ensemble d'alimentation ; ensemble d'alimentation ***caractérisé* en ce que** le piston (18) creux comporte un soufflet métallique déformable et est mobile avec un faible frottement sur une buse (20) de la partie haute pression (1).

2. - Ensemble d'alimentation selon la revendication 1., ***caractérisé* en ce qu'**il comporte un conduit (11), pratiqué dans le corps (2) de la partie haute pression (1), mettant l'intérieur du piston (18) à la pression atmosphérique.

3. - Ensemble d'alimentation selon la revendication *2,* ***caractérisé en* ce que**, un ressort de compression (23), placé à l'intérieur du piston (18), équilibre l'effet de la pression du gaz pour la fermeture de la buse (20) par un clapet (22) disposé dans la tête (19) du piston.

4. - Ensemble d'alimentation selon les revendications 1 à 3, ***caractérisé en* ce que** le dispositif de sécurité haute pression (8) comprend un disque de rupture (9) dont l'éclatement calibré libère le gaz provenant de la bouteille par le collecteur d'échappement (10) à sortie unique.

5. - Ensemble d'alimentation selon la revendication 4, **caractérisé *en ce que*** le dispositif de sécurité basse pression (27) comprend un clapet taré (28) se déplaçant sous l'effet d'une surpression pour permettre l'évacuation du gaz à travers le collecteur (10) à sortie unique.

6. - Ensemble d'alimentation selon la revendication *5, **caractérisé en ce qu'**il* comporte un clapet (12), placé en amont du dispositif de sécurité haute pression (8), limitant la pression résiduelle de la bouteille à un niveau supérieur à celui de la pression de l'air ambiant.

7. - Ensemble d'alimentation selon l'une quelconque des revendications précédentes, ***caractérisé en ce que*** la partie haute pression (1) comporte un robinet (13) commandant le passage, dans la buse (20), du gaz provenant de la bouteille.

8. - Ensemble d'alimentation selon la revendication 7, ***caractérisé en ce que*** le robinet (13), muni d'un volant (15), comporte un doigt (14) coulissant dans un logement du volant pour indiquer l'ouverture ou la fermeture du robinet (13), selon la position rentrée ou sortie du doigt dans le logement.

9. - Ensemble d'alimentation selon l'une quelconque des revendications précédentes, ***caractérisé en ce que*** les matériaux constitutifs de cet ensemble sont compatibles aux contraintes liées à l'oxygène sous haute pression, à la non génération de gaz toxiques en cas d'inflammation, ainsi qu'aux contraintes liées à l'utilisation en milieu aéronautique.

## Claims

1. Gas supply assembly for connection to a high-pressure gas container, comprising a low-pressure part (16) containing a piston (18) and fixed to a high-pressure part (1), each part including a safety device (27, 8) and these two safety devices being interconnected by a venting collector (10) with a single outlet channelling the gas to the outside of the supply assembly, the supply assembly being **characterised in that** the hollow piston (18) has a deformable metal bellows and is movable with low friction on a nozzle (20) of the high-pressure part (1).

2. Supply assembly according to Claim 1, **characterised in that** it includes a conduit (11) formed in the body (2) of the high-pressure part (1), which conduit (11) places the interior of the piston (18) under atmospheric pressure.

3. Supply assembly according to Claim 2, **characterised in that** a compression spring (23) located inside the piston (18) balances the effect of the gas pressure tending to close the nozzle (20) by means of a valve (22) arranged in the head (19) of the piston.

4. Supply assembly according to Claims 1 to 3, **characterised in that** the high-pressure safety device (8) comprises a break disc (9), the calibrated rupture of which releases the gas coming from the bottle through the venting collector (10) with a single outlet.

5. Supply assembly according to Claim 4, **characterised in that** the low-pressure safety device (27) comprises a calibrated valve (28) which moves under the effect of excess pressure to permit venting of gas through the collector (10) with a single outlet.

6. Supply assembly according to Claim 5, **characterised in that** it includes a valve (12) located upstream of the high-pressure safety device (8) and limiting the residual pressure of the bottle to a level greater than that of ambient air pressure.

7. Supply assembly according to any one of the preceding claims, **characterised in that** the high-pressure part (1) includes a tap (13) controlling the entry into the nozzle (20) of the gas coming from the bottle.

8. Supply assembly according to Claim 7, **characterised in that** the tap (13), which is provided with a control wheel (15), includes a finger (14) sliding in a receptacle in the control wheel to indicate the opening or closing of the tap (13), depending on the inserted or retracted position of the finger in the receptacle.

9. Supply assembly according to any one of the preceding claims, **characterised in that** the constituent materials of said assembly are compatible with the stresses associated with oxygen under high pressure, with non-generation of toxic gases in the event of inflammation, and with the stresses associated with utilisation in an aeronautical environment.

## Patentansprüche

1. Gasversorgungsbaugruppe, die dazu vorgesehen ist, an einen Behälter angeschlossen zu werden, der ein unter hohem Druck stehendes Gas enthält, mit einem Niederdruckabschnitt (16), der einen Kolben (18) aufweist und an einem Hochdruckabschnitt (1) befestigt ist, wobei jeder Abschnitt eine Sicherheitsvorrichtung (27, 8) aufweist und diese beiden Sicherheitsvorrichtungen über eine Auslasssammeleinrichtung (10) mit einem einzigen Ausgang, die das Gas zum Äußeren der Versorgungsbaugruppe kanalisiert, miteinander verbunden sind, wobei die Versorgungsbaugruppe **dadurch gekennzeichnet ist, dass** der hohle Kolben (18) einen verformbaren Metallbalg aufweist und mit einer geringen Reibung an einer Düse (20) des Hochdruckabschnitts (1) beweglich ist.

2. Versorgungsbaugruppe nach Anspruch 1, **dadurch gekennzeichnet, dass** sie eine im Körper (2) des Hochdruckabschnitts (1) ausgebildete Leitung (11) aufweist, die das innere des Kolbens (18) auf atmosphärischen Druck bringt.

3. Versorgungsbaugruppe nach Anspruch 2, **dadurch gekennzeichnet, dass** eine Druckfeder (23), die im Kolben (18) angeordnet ist, die Wirkung des Drucks des Gases zum Schließen der Düse (20) mittels eines im Kopf (19) des Kolbens angeordneten Ventilelements (22) ausgleicht.

4. Versorgungsbaugruppe nach den Ansprüchen 1 bis 3, **dadurch gekennzeichnet, dass** die Hochdrucksicherheitsvorrichtung (8) eine Berstscheibe (9) aufweist, deren kalibriertes Bersten das Gas freigibt, das über die Auslasssammeleinrichtung (10) mit einem einzigen Ausgang aus der Flasche kommt.

5. Versorgungsbaugruppe nach Anspruch 4, **dadurch gekennzeichnet, dass** die Niederdrucksicherheitsvorrichtung (27) ein austariertes Ventilelement (28) aufweist, das sich unter der Wirkung eines Überdrucks verlagert, um das Abführen des Gases durch die Sammeleinrichtung (10) mit einem einzigen Ausgang zu ermöglichen.

6. Versorgungsbaugruppe nach Anspruch 5, **dadurch gekennzeichnet, dass** sie ein Ventilelement (12) aufweist, das stromaufwärts der Hochdrucksicherheitsvorrichtung (8) angeordnet ist und den Restdruck in der Flasche auf ein Niveau begrenzt, das höher ist als das des Drucks der Umgebungsluft.

7. Versorgungsbaugruppe nach einem der vorhergehenden Ansprüche, **dadurch gekennzeichnet, dass** der Hochdruckabschnitt (1) einen Hahn (13) aufweist, der das Strömen des aus der Flasche kommenden Gases in die Düse (20) steuert.

8. Versorgungsbaugruppe nach Anspruch 7, **dadurch gekennzeichnet, dass** der Hahn (13), der mit einem Bedienungsrad (15) versehen ist, einen Zapfen (14) aufweist, der in einer Aufnahme des Bedienungsrads gleitet, um den geöffneten oder geschlossenen Zustand des Hahns (13) anzuzeigen, je nachdem, ob sich der Zapfen in der Aufnahme in einer eingeschobenen oder herausgeschobenen Stellung befindet.

9. Versorgungsbaugruppe nach einem der vorhergehenden Ansprüche, **dadurch gekennzeichnet, dass** die grundlegenden Materialien dieser Baugruppe mit den Belastungen kompatibel sind, die mit dem unter hohem Druck stehenden Sauerstoff und damit verbunden sind, dass bei einer Entzündung keine toxischen Gase erzeugt werden, und mit den Belastungen, die mit der Verwendung auf dem Gebiet der Luftfahrt verbunden sind.
